# EUROPEAN PATENT SPECIFICATION

(11) **EP 0 989 188 B1**
(45) Date of publication and mention of the grant of the patent: **07.11.2007**
(21) Application number: 98917133.5
(22) Date of filing: 07.05.1998
(51) Int. Cl.: C12Q 1/00, C12M 1/34

(54) **MICROBIOSENSOR FOR THE CONTINUOUS MONITORING OF CHEMICAL SUBSTANCES IN FLUIDS**
MIKROBIOSENSOR ZUM KONTINUIERLICHEN NACHWEIS VON CHEMISCHEN SUBSTANZEN IN FLÜSSIGKEITEN
MICROBIOCAPTEUR POUR LE MONITORAGE EN CONTINU DE SUBSTANCES CHIMIQUES EN MILIEUX FLUIDES

(30) Priority: 19.05.1997 ES 9701073
(43) Date of publication of application: 29.03.2000
(73) Proprietor: Biosensores, S.L., 12593 Moncofar (ES); Diez-Caballero Arnau, Teofilo, 46004 Valencia (ES)
(72) Inventor: DIEZ-CABALLERO ARNAU, Teófilo, E-46004 Valencia (ES); RODRIGUEZ ALBALAT, Guillermo, E-12540 Villarreal (ES); FERRER FERRER, Cristina, E-12530 Burriana (ES); ESPINAS MARTI, Enrique, E-12593 Moncofar (ES); MONTORO RODRIGUEZ, Sergio, E-46182 Paterna (ES); ERCHOV, Vladimir, E-46021 Valencia (ES); MENDOZA PLAZA, Alejandro, E-46006 Valencia (ES); DIEZ-CABALLERO, Teofilo, Diego, E-46004 Valencia (ES)
(74) Representative: Sanz-Bermell Martinez, Alejandro
(86) International application number: PCT/ES1998/000127
(87) International publication number: WO 1998/053090

(56) References cited:
- EP-A- 0 531 955
- DE-A- 2 163 612
- DE-A- 3 714 612
- DE-A- 3 818 906
- DE-A- 4 332 163
- DE-A- 4 332 165
- DE-A- 19 547 655
- DE-U- 29 513 115
- DE-U- 29 607 093

## Description

### Technological area of this patent.

Chemical and biochemical analysis. Environmental analysis.

System for FIA (Flow injection analysis) with discontinuous flow.

System for automatic analysis with autocalibration.

Application in many areas; environment, microbiological reactors, fermenters, biology, agricultural feeding and industries related to these given areas.

### State of the art

During the last two decades there has been a growing development in the technology of biosensors as a result of the multidiscipline integration between the areas of enzymology, immunology and microbiology and those of sensors and signal transducers. This has allowed the development of biosensor with specific applications (industrial, biomedical or environmental to name a few) that can now compete with the classical analytical technologies. If enzymatic biosensors were to have a special application in the cases of repetitive identification of a molecule/substrate, for which the corresponding enzyme can be found, and if the use of immunobiosensors would allow, in the near future, the specific identification of any simple molecule or compound obtaining their respective antibodies, then microbiosensors could have many applications varying from environmental monitoring to the control of bioreactors.

More recently, the advances in the field of genetic modification of microorganisms are now providing researchers with the elements necessary to design microbiosensors capable not only of detecting and quantifying specific compounds, but also of amplifying the generated signals in the detection, making use of the molecular biology of the modified microorganisms.

Among those configurations most studied until the present day for the design of microbiosensors you have to look at those of microbial membranes in the is technology microorganisms, specific or not, are held in the interior of a membrane and are in contact with the signal transducers of the microbiosensor. The presence of substances that could affect the metabolism of the microbe produce a physical-chemical change in the membrane containing the microbes which is detected by the sensor and if the signal is suitably handled then the substances of interest can be measured. This technology has allowed the appearance on the market of commercial equipment with new and interesting specifications mainly in the environmental area (microbiosensors - BOD like those of Doctor Lange, Medingen or LKG among others) Nevertheless these apparatus that are based on the retention of microorganisms in membranes have intrinsical limitations stemming from the idea and design.

To start with the microorganisms that are fixed to the membrane structure have little or no capacity to grow and are at risk in the presence of certain toxins or sterilising factors which can render the membrane unusable. Also the size and physical/chemical characteristics of the membrane can limit the diffusion of analytes across it and can reduce the concentration margin of the analytes for which the apparatus is capable of generating linear responses (E. Praet, V. Reuter, T. Gaillard, J.-L Vasel. "Bioreactors and biomembranes for biochemical oxygen demand estimation" Trends in analytical Chemistry, vol. 14, n°7, 1995).

Another big area of microbiosensors bases the functional design on the utilisation of a microbial reactor maintained in optimal conditions for microbial activity, into which is added the liquid sample, to be monitored, directly into the reactor, registering the signal produced by the transducer. In some of the variants of this type of microbiosensor you can adjust the volume, dilution or the flow of injection of the sample by means of automatic systems of feedback of the signal generated so that a predetermined value is maintained. Good examples of these microbiosensors are the respirometers like Rodtox (P.A. Vanrolleghem, et al. "An on-line Respirographic Biosensor for the Characterization of Load and Toxicity of Wastewater", J. Chem. Tech. Biotechnol. 1994, 59,321-333) and Stip. These types of microbiosensors are being used at the moment with specific applications (mainly BOD and toxicity) with satisfactory results but its design has functional limitations in many applications of interest. This is due to the overall change in the parameters of the microbiological reactor (concentration of microbes, functional change in the microorganisms, etc.) on introducing the sample to be analyzed into the reactor, showing that on occasions there may be considerable concentrations of toxic chemical products capable of altering, over long periods of time, the overall response of the microorganism sensors. Consequently i is difficult to maintain a base line of steady response making it impossible to analyze samples of high toxicity or, if you risk analyzing these samples, reversibly or irreversibly damaging the microbiosensor.

The invention about to be described has changed the concept and design of microbiosensors making it possible to overcome a large number of limitations that they had before. Among the innovations that it has and the advantages it has over the actual technology of microbial membranes, in summary, are the following:
a) continuous availability of microorganisms when using a microbiological reactor with controlled growth parameters which allows the monitoring of samples of high toxicity discarding the microorganisms used in the minireactor or reaction unit after every measurement.
b) the reaction unit or minireactor for the analysis is an independent compartment from the mother reactor in which there is the continuous cultivation of microorganisms, in the minireactor the samples for analysis are injected without interfering with the action of the continuous cultivation of cells.
c) it eliminates the membrane which physically separates the medium to be analyzed and the transducer, thus allowing the amplification of the range of the signal and reducing drastically the time necessary to reach a steady signal.
d) The introduction of the reference sample before and after the injection of the problem sample into the reaction unit makes it possible to systematically autocalibrate the apparatus and obtain a stable base line of reference.
e) Discarding the contents of the minireactor that monitors the sample after every cycle makes it possible to inject into it a high ratio of: (volume of problem sample) / (total volume) which allows the detection of certain chemical compounds in small concentrations in the problem sample or with little capacity to generate a signal in the transducer.

It must be emphasized that the invention described does not refer to require a certain type of microorganism. The technology of the invention correspond to a microbiosensor that is made up of automatic devices, reactors, and minireactors transducers, PLC's, PC, thermostat systems etc. which makes possible its use in several applications, utilising specific microorganisms in each case, and not only those available at the time but those also that could be obtained in the future. In whichever of the possible applications of this patent, the advantages described in the previous paragraph will be obtained in whole or in part in comparison with the technology in use today.

### Description of the invention

The technology and innovations arising from this patent, can be applied to a variety of types of microbiosensors, as much in the cultures used, pure or mixed, as the analysis monitored, specific molecules or in specific substrates, or by the physical-chemical signal generated and the transducer used to quantify. As examples to explain the wide usage of the developed technology references will be made to a/ pure microbial strains, natural or genetically modified, that allow the monitoring of specific molecules using, for example optoelectronic signal transducers and h/ mixed microbial cultures capable of metabolising or altering their metabolism when exposed to a wide variety of molecular species (analytes) present in the problem sample (example-BOD).
a/ As a reference to this application is a short description of the use of genetically modified microorganisms into which have been inserted a determined genetic sequence via DNA recombinant technology. This sequence is made up of the genes called LUX which code the synthesis of the enzyme luciferase, which catalyses the oxidation reaction associated with the emission of one photon of light with a wavelength of 490 nm. This enzyme comes from a procaryotic organism (Philip J. Hill. Stephen P. Denyer." Rapid Assays based on in vivo luminescence" Microbiology Europe, 16, May/June 1993), (Robert S. Burlage." Living Biosensors for the Management and Manipulation of Microbial Consortia" Annual Review Microbiol., 48. 81-104).

Associated to the same promotor (region of the genome where the transcription of a gene occurs) are inserted the genes which code for the metabolic route of interest. When the specific metabolite to be measured (toxic) is present in the medium, the promotor of this genetic sequence is activated and transcribes both the genes that code for luciferase and those that code for the enzymes that catalyze the degradation of the said metabolite (Jorma Lampinen, Marko Virta. "Use of Controlled Luciferase Expression To Monitor Chemicals Affecting Protein Synthesis." Applied and Environmental Microbiology, Aug. 1995, p. 2981-2989).

The analytes-molecules can be organic compounds that are considered toxic in the environment, like organochloro compounds. The bacteria metabolise these toxins, emitting light in the process, which can be detected and quantified, relating to the toxin present in the medium (S. Burlage A. Palumbo." Bioluminescent Reporter Bacteria Detect Contaminants in Soil Samples" Applied Biochemistry and Biotechnology, vol.45/46). The most innovative bacterial strains incorporate a constitutive control which consists of the insertion of a gene coding for the synthesis of a eucaryotic luciferase into the bacterial chromosome, the expression of which is constitutive. And so you can detect the continuous emission of light at 560 nm as an indicator of the good metabolic state of the biomass and in addition a light at 490 nm when a specific toxin is present in the medium (Angel Cebolla, F. Ruiz-Berraquero." Stable Tagging of Rhizobium meliloti with the Firelly Luciferase Gene for Environmental Monitoring" Applied and Environmental Microbiology,Aug. 1993, p.2.511-2519) (Wood Kv. Gruber MG." Transduction in Microbial Biosensors Using Multiplexed Bioluminiscence. "Biosensors and Bioelectronics 11(3):207-214,1996), (Gu MB. Dhurjati PS." A Miniature Bioreactor for Sensing Toxicity using recombinant bioluminiscent Escherichia coli Cells". Biotechnology Progress.12(3):393-397,1996); b/ to go deeper in the function and technological aspect of this patent a detailed example of the application of this patent to monitor on-line the Biochemical Oxygen Demand (BOD).

### A working example of the application of the invention in the on-line monitoring of the Biochemical Oxygen Demand (BOD)

A microbiosensor system is presented for the on-line monitoring of chemical substances in fluids. The use of the system in the determination of the Biochemical Oxygen Demnand will be described as a particular case and example of an application of the presented invention. The measurement of the Biochemical Oxygen Demand (BOD) is fundamental in controlling the function of the WWTP (Waste Water Treatment Plant) and for the monitoring and observation of ecosystems. It is an analytical parameter that measures the oxygen used by microorganisms for the biochemical degradation of organic material contained in a sample during a specific incubation period at a given temperature. This information allows the adjustment of the oxygen needs of the biological reactor which is vital for the function of the WWTP.

The traditional ways of measuring the BOD require and incubation time of 5 days in the laboratory, which mean that the result obtained did not reflect the pollution of the water that needed to be treated at that moment in the WWTP.

As a consequence these analytical systems do not have much use in operative control processes that require sample quantification in shorter times. It is also an analysis with a considerable number of sources of variability in the results.

The BOD microbiosensor equipment, as an example of the application of the invention, is based on advanced and original technologies compared to other apparatus with the same purpose which are on the market today, and makes it possible to obtain a reading of BOD in liquid samples of diverse composition in only 15 minutes. It is then possible to:
a/ obtain up to date information about the performance of a WWTP or of the liquid volume analyzed.
b/ if necessary take instant correct measurements, being able to assess in a short time the effect produced by the applied measurements.
c/ save energy used for aeration, one of the costly processes of the WWTP
d/ centralise the monitoring of one or many WWTP by means of the remote capture nf data.
e/ identify and analyze the presence of certain toxins in the liquid samples analyzed.
f/ determine the BOD even in liquid samples with elevated levels of toxins.
g/ autocalibrate every cycle, measuring the analytical response of the apparatus using internal reference samples.
h/ monitor the BOD of open systems in a wide variety of environmental temperatures.

In the following notes the function and components of the microbiosensor apparatus for the monitoring of chemical substances in fluids will be examined, in the specific case of the on-line determination of the BOD in water.

The configuration of the microbiosensor for the continuous monitoring of chemical substances in fluids will now be described.

The microbiosensor is made up of an integrated system and includes
a/ units for the continuous growth of microorganisms.
b/ microbiological reaction units.
c/ transducer component (dissolved oxygen sensor in the case of the BOD)
d/ a programmed hydraulic circuit for the handling of liquids.
e/ microprocessor for the control of the analytical process and the acquisition of the signal.
f/ PC device for the treatment of the data and communication with the PLC (microprocessor).
g/ integrated thermostabilisation devices.

The microbiosensor should be able to use the apparatus. This systems is composed of peristaltic pumps to handle the samples to be analyzed, from the specified points in the WWTP into the apparatus and a tangential filter that eliminates the solid suspensions that could interfere with the measurement of the BOD.

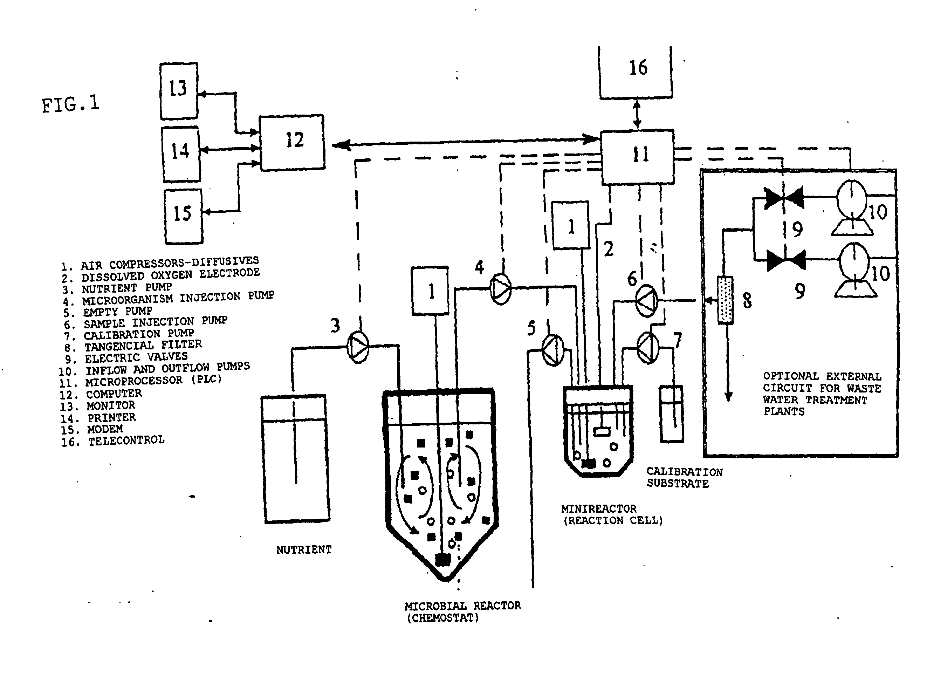

Figure 1 shows the layout of the microbiosensor. The first stages of the functioning of the microbiosensor for the continuous monitoring of chemical substances in fluid arc laid out as follows:

In the reaction unit is situated the transducer component. In this case a small dissolved oxygen sensor which measures the reduction of dissolved oxygen due to the metabolisation of organic material in the sample

In each cycle predetermined quantities of the following substances are added:
a/ the corresponding aliquot of the suspension of microorganisms of predetermined quantity are transferred In every cycle into the minireactor from the mother reservoir (RH).
b/ the reference sample substance, whose corresponding value of the analytical variable is measured and this allows the testing of the measuring system. In each cycle an autocalibration of the microbiosensor takes place by means of an automatic addition of a predetermined amount of the reference sample (P) before ana after the addition of the samples to be analyzed. In this way in every cycle the toxic effect that the sample analyzed could have on the microorganisms used in the measurement can be determined by means of the comparison between the corresponding signals of the reference samples before and after the addition of the problem samples into the reaction cell.
c/ The water samples to be analyzed that are previously driven by peristaltic pumps to the microbiosensor equipment within a certain time that allows for the dead volume in the conduction tubes to be referred, from the place of the taking of the sample to the microbiosensor BOD, ensuring that the samples to be analyzed are fresh.

The liquid for washing consists of a cleaning solution in the corresponding reservoir within the microbiosensor apparatus and allays for the cleaning of the minireactor and the active surfaces of the transducers after every cycle once the cycle has finished.

The washing step begins with the emptying of the contents of the minireactor followed by the addition of the cleaning solution, and once full the solution is kept in the cell and is watered by a method of bubbling for a set time, this is followed finally by the emptying of the cell. The automatic addition of the cleaning liquid occurs by means of a jet of liquid over the active surfaces of the transducer component, which increases the period of time that the microbiosensor, still with the same response capacity in the same conditions, can remain autonomous and so by reducing drastically the needs of maintenance and manipulation by human intervention.

The oxygen needed for the reaction is continually supplied to the minireactor via a preset air primp and fitted with a fitter which captures the solid particles present in the air (1). When the sample that is injected into the reaction unit contains organic material, the microorganisms metabolise it, producing a concomitant consumption of dissolved oxygen.

The components of the microbiosensor apparatus for the continuous monitoring of chemical substances in fluids are as follows: the fluid systems: An internal fluid system, that consists of a system capable of driving, regulating and channelling the different fluids towards the microreactor, that all integrate into flow system. This system consists of the following parts: a driving system that is made up of peristaltic pumps (3,4,5,6,7) with each pump driving a different fluid, distribution tubes that are flexible; through which circulate the different fluids, a three way electric value that controls the passage of the sample into the cell and a aireaction dence made up of compressors and easupped the corresponding distribution tubes so as to aerate the microorganisms in suspension: An external fluid system: An external hydraulic circuit used for the taking of the sample has filtration (8) dences for the elimination of solids in suspension that have no analytical value or that could also damage the internal components of the microbiosensors, especially in the case of servage.

These dences are situated first at the point of collection of the sample and at a point just before the entrance of the sample into the microbiosensors apparatus. In the last case, the device is made up of an on line tangential filter (8) so that the differences in pressure generated along the filter allow for the production of a filtrate free of solids in suspension. This flow of filtered sample is directed to the electrovalve, which in accordance with the analytical protocol allows the entrance of the filtered sample, in a quantity defined by the protocol, into the minireactor having already assured the remoral of the dead volume of previous samples so as to avoid the contamination between samples of consecutive cycles.

Thermostability. At thermostability systems that allows the simultaneous maintenance of an adequate temperature for microbial growth (fixed between 25 and 50°C) and an adequate temperature (2- 8°C) for the conservation of the reaction media during a long period of time. The systems is made from two dences.

A cold/hot thermostabilisation dence, that consists of a compartment with a thermostat which contains the components and liquid medium of the microbiosensor equipment. The system maintains the compartment at refrigeration temperature for the maintenance of a stable reactive medium during long periods of time.

A thermostabilisation dence of the reaction unit, consisting thermally protected compartment situated within the cold/hot thermostabilisation device adjustable thermostat is incorporated. Both systems have sensors which allows the visual control of the temperature, given its importance, as the temperature influences the dissolved oxygen concentration and also the permeability to oxygen of the membrane of the electrode with which the amount of oxygen is measured.

The microbiological system of the microbiosensor apparatus consists of a in whose of the microorganisms is continually controlled by adjustment of their growth parameters (pH, temperature, dilution, aeration and feeding). Independent of the starting innocuous system is capable of maintaining the concentration and metabolic activity of the microorganisms practically constant within the thermostat controlled reaction unit. Their metabolism is sensitive to the changes in chemical substances.

The reaction unit is contained within the corresponding thermostatically controlled device and is made up of the following components:

A mother reservoir (RH) is made up of a tank which contains the microorganisms in suspension and whose concentration and metabolic activities are maintained practically constant via the control of: a/the systematic refeeding with a predetermined feeding medium, situated in a reservoir equipped with a ultraviolet lamp which stops or controls the growth of microbial contaminants and b/ the maintenance of the preestablished microbial growth parameters (temperature, pH, dilution, aeration)

Structures of similar density to the liquid medium, hollow or with pores long enough to allow their colonisation by microorganisms are introduced into the interior of the mother reactor and they serve as a concentrated innocuous to accelerate the functional recovery of the continuous cultures. In cases of changes of apparatus or functional accidents whatsmore these structures favour the dissolving of oxygen in the suspension of microorganisms because they increase the time of contact between the air/liquid. In each cycle of the microbiosensor an aliquot of microorganisms is passed into the reaction cell (MR).

The minireactor (MR) is the dence where the component of the reaction are added and mixed, where the reaction takes place and is monitored using the corresponding transducer component. In the minireactor interact the microorganisms with the components of the sample that are to be analyzed, the time and temperature are determined by the specific analysis protocol and are specified by the control system of the analytical process.

The transducer component in case of the BOD is a Clark type (2) dissolved oxygen electrode, capable of measuring the oxygen concentration in the reaction mixture. The main job of the oxygen electrode is to determine the partial pressure of oxygen in liquids in accordance with the principles of Clark. The process of measurement is based on the separation of the sample and the electrode by a permeable membrane.

The reduction of oxygen at the working electrode is by the following reaction

O₂ + 2H₂O + 4e⁻ --> 4OH⁻

The oxidation of the reference electrode results from the consumption of electrons.

The steps between the fitting of the sensor and setting it working are shown here: the fixing in place and stretching of the membrane, the addition of the electrolyte, assembly of the hood over the sensor and the turning on of the fixed sensor.

The control system of the analytical process consists of a computer (PC) (12) that continually supervises the operation of a programmed microprocessor (PLC) (11), which carries out the instructions of the specific analysis protocol controlling between other parameters the following: volumes and sequences of the taking of the components of the process, reaction times before the reading of results, obtaining the signal generated by the transducer, an autocheck of the system and finally the calculation and presentation of results.

The computer (PC) carries out the complex processing of the signals from the transducers and permits the modification of the controls carried out by the programmable microprocessor depending on the needs of the measurements or the conditions of the function of the system. The incorporation modems into the computer system allows the microbiosensor equipment to be remote controlled by means of a telephone connect (16), thus making it possible to centralise the control of various microbiosensor situated at different WWTP. In addition, the apparatus has a monitor (13) and a printer (14).

As an example, the control software and acquisition of data from the microbiosensor of the present invention, in the particular case of the BOD, will be described.

The BOD microbiosensor is controlled by specialised software. The apparatus of the microbiosensor includes a PC computer and a microprocessor (the PLC). The control programmes implement the following functions:
the graphical design of a diagram showing the function of the devices with respect to time for a specific protocol.
   - control of the function of the electric devices of the fluid circuit.
   - acquisition of the signals from the sensor(s).
   - display of the obtained data on the screen of the computer.
   - -preliminary treatment of the data.
   - storage of the data.

The software of the microbiosensor includes two main programmes and various additional programmes (for the remote control of the apparatus, to construct graphs, and so on). The programme of the PLC microprocessor allows for the control of the function of the apparatus independent of the PC computer, whilst the programme of the PC allows for the receiving, storing and analysis of the data.

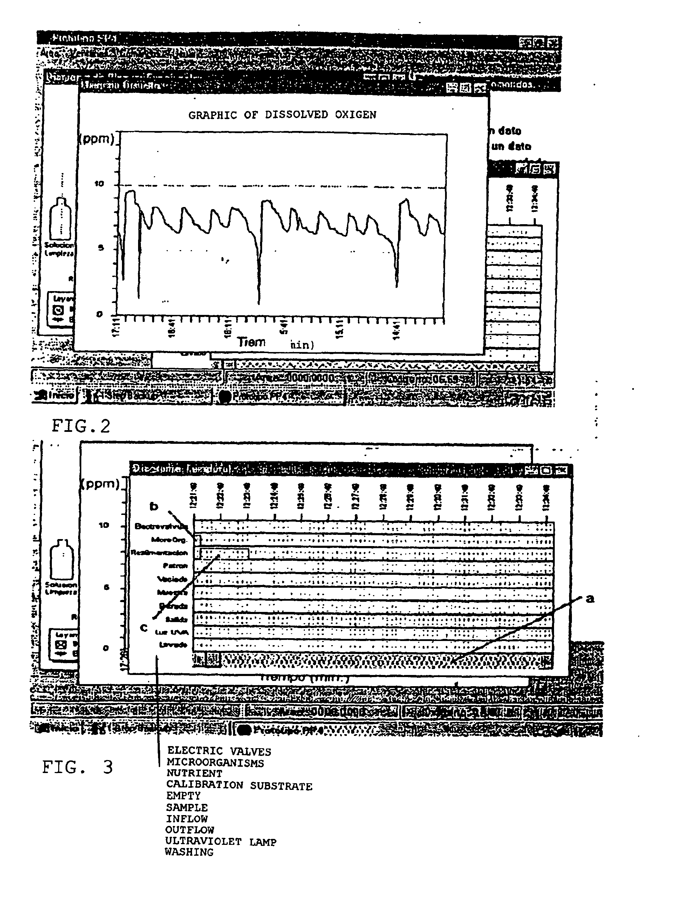

The programme of the PC computer receives every 30 seconds the data from the PLC microprocessor and stores them in a file on a hard disk. At the same time, the programme displays these data in graphical form and analyses the changes observed during the cycle. This graph of dissolved oxygen (fig.2) is shown in a window where the operator can note any point of interest and calculate the areas under the peaks that correspond to the respirogram responses of the microbiosensor to the sample.

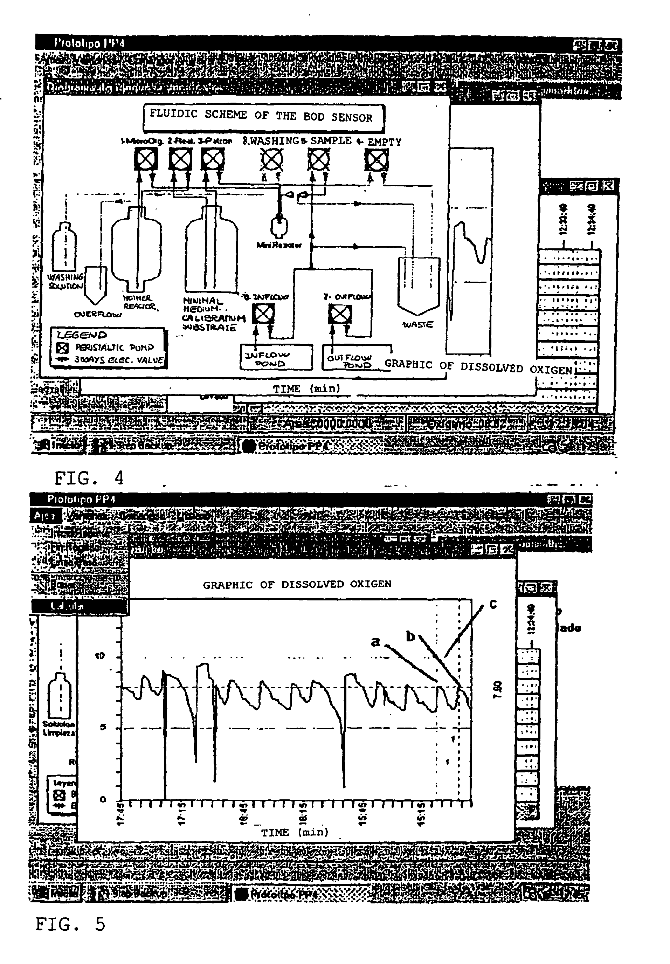

The PC programme prepares two screens more: one called diagram of time and the other one diagram of the function of the apparatus. The diagram of time (fig.3) allows you to observe the function of the cycle using the graphs of activation and inactivation of the devices in the fluid circuit. The function diagram (fig.4) is a window that helps to the better understanding of the function of the microbiosensor and allows the control of the electrical devices of the apparatus (the peristaltic pumps and the electrovalves). The hardware of the PLC microprocessor is designed so that the exchange of messages between the microprocessor and the PC computer can occur without interrupting the programme of the microprocessor.

The function diagram (fig.4) represents the structure of the fluid circuit of the microbiosensor. It includes the minireactor, the mother reactor, the peristaltic pumps, liquid circuits, electovalves, etc. This diagram permits the control of the peristaltic pumps and electrovalves from the screen of the PC computer. Any pump or electrovalve can be activated simply by moving the cursor onto its image and pushing the left key of the mouse. The image of the pump changes, if you press again the image of the activated pump it stops. In the same way the electrovalve can be activated / inactivated.

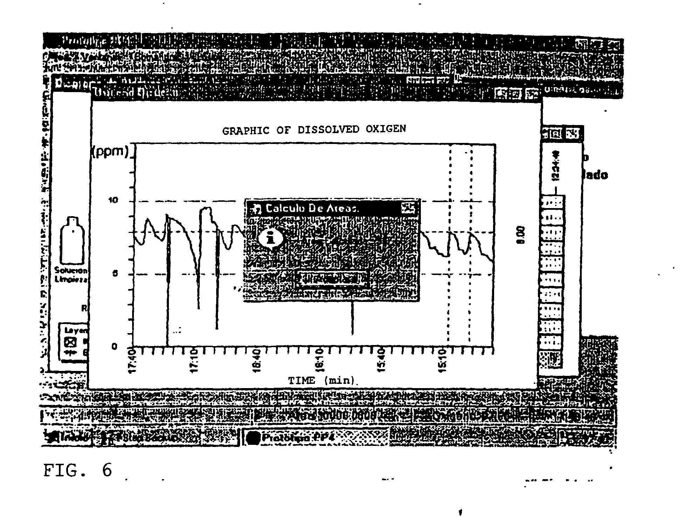

The diagram of dissolved oxygen shows the up to date data obtained during the cycle. The data represents the 600 last dissolved oxygen measurements (taken as often as required). Normally the peaks of sample measurements and the reference sample peaks appear on the diagram (references, **fig.2).** The area of the peaks represent the biochemical oxygen demand (BOD). When a new peak appears, the new area is calculated automatically and the result of the calculation is shown in the bottom part of the window in the information bar. It is also possible to calculate the area of any peak on the graph by using the "Area" command in the menu. By using the "Start Recording", "Finish Recording" and "Base line" commands (fig.5) it is possible to mark the limits for the integration of the respirometric graph (moving the cursor to the corresponding points of start and end of integration and the base line from which the area can be calculated; points a, b and c in fig.5). By using the "Calculate" command the marked area will be calculated and the result will be shown on the screen (fig.6). The diagram of time shows where in the analytical cycle the apparatus is. The state of the peristaltic pumps (Microorganisms, Refeeding, Reference, Empty, etc.)is seen as an activated unit (the upper signal) if the pump is activated, or at zero if the pump is switched off.

The apparatus can also be controlled by using the menus "Windows", "Commands", "User". With the menu "Windows" the operator chooses one of the three windows to control the apparatus (Dissolved Oxygen, Diagram of Time or Boxes Diagram). By pressing one of the items in the "windows" menu, the corresponding window will be shown in front of the others and the operator can then use the diagram. The "User" menu serves to regulate the level of access to the commands (for example, a supervisor can change the properties of the cycle but another operator will not have access to this command). The "Command" menu serves to activate mini cycles in the fluid circuit.
- The command "Stop the Cycle" is carried out at a programmed time and includes the activation of various pumps (those of washing and emptying).
- The command "Change Cycle Parameters" serves to change the function times of the cycle.
- The command "Microorganisms" activates the microorganism pump and, after that, the pump for the refeeding of the mother reactor.
- The command "Reference Sample" activates the reference sample pump after u predetermined time.
- The command "Inlet Sample" starts the part of the cycle involved in the taking of a sample from the entrance to the WWTP. At first, the external pump for the sample is activated at the entrance to the WWTP allowing the sample to reach the apparatus. After a time, both the internal peristaltic pump and the electrovalve for the injection of the sample into the minireactor are activated.
- The command "Outlet Sample" starts the part of the cycle involved in the taking of a sample from the outlet of the WWTP which follows the same pattern as the command described above, for the taking of a sample from the inlet of the WWTP.

## Claims

1. A method for continuous monitoring the presence of chemical substances in fluids, said continuous monitoring being carried out in consecutive cycles using a microbiosensor **characterized in that** it comprises the steps of:
a/ the culture in a chemostat placed into a thermostatic compartment of a suspension of microorganisms whose metabolism is modified proportionally to the concentration of chemical substances of interest present at the liquid sample to be analysed;
b/ the extraction of an aliquot of the cultured microorganisms from the chemostat for each of the analytical cycle into a separate compartment;
c/ the metabolic activity and concentration of the cultured microorganisms in the chemostat are maintained practically constant by the injection of a liquid nutrient (feeding-medium) for the systematic refeeding of the microbial suspension into the chemostat;
d/ the growth of the cultured microorganisms in the suspension and also into the hollow or porous free structures placed into the chemostat to create a micro adequate, environment for the colonisation of the microorganisms.
e/ the injection of a forced air flux into the microbial suspension;
The measurement, being carried out by
g/ injecting an extracted microbial aliquot from the chemostat into a separate compartment called reaction cell, in each analytical cycle ;
h/ injecting a reference sample with specific composition into the reaction cell, for the auto calibration of the system in each analytical cycle;
i/ obtaining a physical-chemical signal generated in the microbial metabolism by the chemical substances present in the reference sample;
j/ pre-treating the sample to be analysed by the tangential filtration to remove suspended solids;
k/ injecting the sample to be analysed into the reaction cell;
l/ obtaining a physical-chemical signal generated in the microbial metabolism by the chemical substances present in the filtered sample;
m/ transducing the physical-chemical signals generated by the presence of chemical substances of interest into analytical parameters by the use of a sensor;
n/ comparing between signals generated by the sample and the signals generated by the reference sample, obtained in the reaction cell by the use of a sensor;
o/ Discarding the contents of the reaction cell, after each analytical cycle.

2. A method for continuous monitoring the presence of chemical substances in fluids, according to claim 1, **characterized in that** the liquid nutrient used in the step (d) is pretreated by sterilisation using a system based on ultraviolet lamp.

3. A method for continuous monitoring the presence of chemical substances in fluids, according to claim 1 to 2, **characterized in that** there are used a system to clean the reaction cell, the sensors, and the liquid circuits between successive analytical cycles.

4. A method for continuous monitoring the presence of chemical substances in fluids, according to claims 1 to 3, **characterized in that** the Biochemical Oxygen Demand (BOD) can be determined in the reaction cell using the extracted microbial aliquot from the chemostat and a dissolved oxygen electrode placed in the reaction cell.

5. An apparatus (Figure 1) for continuous monitoring the presence of chemical substances in fluids for carrying out the method of the claim 1, which comprises:
a/ a system for the continuous culture of the used microorganisms, comprising:
• a chemostat placed into a thermostatized compartment
• a reservoir of sterilised liquid nutrient into a thermostatized compartment
• a device for the forced air flux into the chemostat
• a device for the independent thermostatization of the chemostat
• a device for the independent thermostatization of the liquid nutrient
• devices for the injection of the liquid nutrient and for the microbial aliquot extraction.
b/ a system for the analytical measurements based on the cultured microorganisms, comprising:
• a reservoir of a reference sample (calibration substrate) placed in a thermostatized compartment
• a reaction cell placed in a thermostatized compartment
• a device for the independent thermostatization of the reaction cell
• a device for the forced air flux of the reaction cell
• a device for the independent thermostatization of the reservoir of the reference sample
• a taking sample circuit with a tangential filter
• devices for the injection of the cultured microorganisms, reference sample, and filtered sample.
c/ a system to clean liquid circuit.

6. An apparatus for continuous monitoring the presence of chemical substances in fluids, according to claim 5, **characterized in that** the reaction cell includes specific sensors for the detection of physical-chemical, signals generated in the microbial metabolism, by the chemical substances of interest present in the sample sample.

7. An apparatus for continuous monitoring the presence of chemical substances in fluids, according to claim 5, **characterized in that** there is used a system to clean the reaction cell, the sensors, and the liquid circuit, which comprises a reservoir of the washing liquid, placed in a thermostatized compartment.

8. An apparatus for continuous monitoring the presence of chemical substances in fluids, according to claim 5, which comprises a software for the integration and treatment of the electrical signals generated by the sensors.

9. An apparatus for continuous monitoring the presence of chemical substances in fluids, according to claim 5, which comprises a microprocessor and its software to the automated control of the apparatus, allowing the selection of the parameters corresponding to the analytical protocol by the user for specific applications.

10. An apparatus for continuous monitoring the presence of chemical substances in fluids; according to claim 5, which comprises a software and a modem for the data transmission through telephone line and for the control of the parameters of the analytical protocol from a remote computer.

## Patentansprüche

1. Verfahren zum kontinuierlichen Nachweis von chemischen Substanzen in Flüssigkeiten, erfolgend in aufeinander folgenden Zyklen, durch Benutzung eines Mikrobiosensors, charakterisiert durch Folgendes:
a/ die Kultur in einem Chemostat, befindlich in einem thermostatischen Kompartiment einer Emulsion von Mikroorganismen, deren Stoffwechsel sich proportional zu der Konzentration von den chemischen Substanzen von Interesse in der zu analysierenden flüssigen Probe modifiziert;
b/ der Entzug eines Aliquot des kultivierten Mikroorganismus aus dem Chemostat in ein abgesondertes Kompartiment, in jedem zu analysierenden Zyklus;
c/ die quasi konstante Aufrechterhaltung der Stoffwechselaktivität und der Konzentration der kultivierten Mikroorganismen durch die Injizierung eines flüssigen Nährstoffes (Nährmedium) zur systematischen Rückkopplung der mikrobischen Emulsion in dem Chemostat;
d/ das Wachstum der kultivierten Mikroorganismen in der Emulsion und auch in den freien hohlen oder porösen Strukturen, angeordnet im Chemostat, zur Schaffung einer adäquaten Mikroumwelt zur Kolonisierung von Mikroorganismen;
e/ die Injizierung eines künstlichen Luftstroms in die mikrobische Emulsion;
Die Messung wird ausgeführt durch:
g/ die Injizierung eines extrahierten mikrobischen Aliquot aus dem Chemostat in ein abgesondertes Kompartiment, genannt Reaktionszelle, in jedem analytischen Zyklus;
h/ die Injizierung eines Vergleichsmusters mit einer spezifischen Zusammensetzung, zur Autokalibrierung des Systems, in jedem analytischen Zyklus
i/ die Erlangung eines physikalisch-chemischen Signals, im mikrobischen Stoffwechsel durch die chemischen Substanzen generiert, die im Vergleichsmuster enthalten sind;
j/ die Vorbehandlung des zu analysierenden Musters durch Tangentialfiltration um die Feststoffe aus der Emulsion zu eliminieren;
k/ die Injizierung des zu analysierenden Musters in die Reaktionszelle;
l/ die Erlangung eines physikalisch-chemischen Signals, im mikrobischen Stoffwechsel durch die chemischen Substanzen generiert, die im filtrierten Muster enthalten sind;
m/ die Umsetzung der physikalisch-chemischen Signale, die durch die Anwesenheit der chemischen Substanzen von Interesse generiert werden, in analytische Parameter, erfolgend durch den Einsatz eines Sensors;
n/ der Vergleich der erhaltenen Signale aus der Probe bzw. dem Vergleichsmuster, die in der Reaktionszelle mittels eines Sensors erlangt werden;
o/ die Abführung der Inhalte der Reaktionszelle nach jedem analytischen Zyklus.

2. Verfahren zum kontinuierlichen Nachweis von chemischen Substanzen in Flüssigkeiten, nach Anspruch 1, wobei der flüssige Nährstoff aus Etappe (c) durch Sterilisation vorbehandelt wird, indem ein auf ultra-violettem Licht basierendes System genutzt wird.

3. Verfahren zum kontinuierlichen Nachweis von chemischen Substanzen in Flüssigkeiten, nach den Ansprüchen 1 bis 2, wobei ein System genutzt wird, um die Reaktionszelle, die Sensoren sowie die liquiden Kreisläufe zwischen den aufeinander folgenden analytischen Zyklen zu säubern.

4. Verfahren zum kontinuierlichen Nachweis von chemischen Substanzen in Flüssigkeiten, nach den Ansprüchen 1 bis 3, wobei der Biochemische Sauerstoffbedarf (BOS) in der Reaktionszelle bestimmt werden kann, durch die Nutzung des extrahierten mikrobischen Aliquot aus dem Chemostat und einer Sauerstoffelektrode, die in der Reaktionszelle platziert wird.

5. Eine Vorrichtung (Figur 1) zum kontinuierlichen Nachweis von chemischen Substanzen in Flüssigkeiten um die Methode aus Patentanspruch 1 zu vollziehen, welche umfasst:
a/ ein System zur kontinuierlichen Kultivierung von Mikroorganismen, das umfasst:
• ein Chemostat, welches sich in einem thermostatischen Kompartiment befindet;
• ein Reservoir mit sterilisierten flüssigen Nährstoffen in einem thermostatischen Kompartiment
• eine Vorrichtung zur künstlichen Belüftung des Chemostats
• eine Vorrichtung zur unabhängigen Thermostatisierung des Chemostats
• eine Vorrichtung zur unabhängigen Thermostatisierung des flüssigen Nährstoffes
• Vorrichtungen zur Injizierung des flüssigen Nährstoffes und zur Extrahierung des mikrobischen Aliquot.
b/ ein System für die analytischen Messungen, die auf den kultivierten Mikroorganismen basieren, das umfasst:
• ein Reservoir eines Vergleichsmusters (Abgleichsubstrat), welches sich in dem thermostatischen Kompartiment befindet;
• eine Reaktionszelle, welche sich in dem thermostatischen Kompartiment befindet;
• eine Vorrichtung zur unabhängigen Thermostatisierung der Reaktionszelle
• eine Vorrichtung zur künstlichen Belüftung der Reaktionszelle
• eine Vorrichtung zur unabhängigen Thermostatisierung des Reservoirs an Vergleichsmustern
• ein Kreislauf zur Musterentnahme mit einem Tangentialfilter
• Vorrichtungen zur Injizierung von kultivierten Mikroorganismen, Vergleichsmustern und filtrierten Mustern.
c/ ein System zur Säuberung des flüssigen Kreislaufes.

6. Eine Vorrichtung zum kontinuierlichen Nachweis von chemischen Substanzen in Flüssigkeiten, nach Anspruch 5, wobei die Reaktionszelle spezifische Sensoren zum Nachweis von physikalisch-chemischen Signalen umfasst, die im mikrobiologischen Stoffwechsel durch die sich im Muster befindlichen chemischen Substanzen von Interesse ausgelöst werden..

7. Eine Vorrichtung zum kontinuierlichen Nachweis von chemischen Substanzen in Flüssigkeiten, nach Anspruch 5, wobei ein System zur Säuberung der Reaktionszelle, der Sensoren und des flüssigen Kreislaufes verwendet wird, welches ein Reservoir an Reinigungsflüssigkeit beinhaltet, welches sich in dem thermostatischen Kompartiment befindet.

8. Eine Vorrichtung zum kontinuierlichen Nachweis von chemischen Substanzen in Flüssigkeiten, nach Anspruch 5, welche eine Software zur Integration und Verarbeitung der elektrischen Signale umfasst, die durch die Sensoren generiert werden.

9. Eine Vorrichtung zum kontinuierlichen Nachweis von chemischen Substanzen in Flüssigkeiten, nach Anspruch 5, welche zur automatischen Kontrolle des Apparates einen Mikroprozessor und seine Software umfasst, die dem Verwender die Auswahl der entsprechenden Parameter für das analytische Protokoll erlaubt, zum Zwecke spezifischer Anwendungen.

10. Eine Vorrichtung zum kontinuierlichen Nachweis von chemischen Substanzen in Flüssigkeiten, nach Anspruch 5, welche eine Software und ein Modem zur Datenübertragung via Telefon und zur Kontrolle des analytischen Protokolls durch einen Ferncomputer umfasst.

## Revendications

1. Méthode pour le monitorage en continu de la présence de substances chimiques dans des fluides, ce monitorage en continu étant menée à bien par cycles consécutifs en utilisant un micro bio-senseur, **caractérisé par le fait qu'**il comprend les étapes suivantes :
a/ la culture dans un chemostat situé dans un compartiment thermostatique, d'une suspension de microorganismes, dont le métabolisme est modifié proportionnellement à la concentration des substances chimiques intéressantes présentes dans le liquide échantillon devant être analysé;
b/ l'extraction, à partir du chemostat, d'une partie aliquote des microorganismes cultivés pour chaque cycle analytique, dans un compartiment séparé;
c/ l'activité métabolique et la concentration des microorganismes cultivés dans le chemostat sont maintenues pratiquement constantes, grâce à l'injection d'un liquide nutriment (moyen d'alimentation) pour la réalimentation systématique de la suspension microbienne dans le chemostat;
d/ la croissance des microorganismes cultivés dans la suspension ainsi que dans les structures libres creuses ou poreuses disposées dans le chemostat pour créer un micro environnement adéquat à la colonisation des microorganismes;
e/ l'injection d'un flux d'air forcé dans la suspension microbienne;
La mesure étant menée à bien à travers :
g/ l'injection d'une partie aliquote microbienne extraite du chemostat vers un compartiment séparé, nommé cellule de réaction, lors de chaque cycle analytique;
h/ l'injection d'un échantillon de référence avec une composition spécifique pour l'auto calibrage du système, lors de chaque cycle analytique;
i/ l'obtention d'un signal physico-chimique généré dans le métabolisme microbien par les substances chimiques présentes dans l'échantillon de référence;
j/ le prétraitement de l'échantillon à analyser par la filtration tangentielle pour éliminer les solides en suspension;
k/ l'injection d'échantillon à analyser dans la cellule de réaction;
l/ l'obtention d'un signal physico-chimique généré dans le métabolisme microbien par les substances chimiques présentes dans l'échantillon filtré;
m/ la transduction des signaux physico-chimiques générés par la présence des substances chimiques intéressantes dans les paramètres analytiques à travers l'utilisation d'un senseur;
n/ la comparaison entre les signaux générés par l'échantillon et les signaux générés par l'échantillon de référence obtenu dans la cellule de réaction à travers l'utilisation d'un senseur;
o/ décharge du contenu de la cellule de réaction, après chaque cycle analytique.

2. Une méthode pour le monitorage en continu de la présence de substances chimiques dans des fluides, selon la revendication 1, **caractérisée par le fait que** le liquide nutriment utilisé au cours de l'étape (d) est prétraité par stérilisation en utilisant un système basé sur une lampe à ultraviolets.

3. Une méthode pour le monitorage en continu de la présence de substances chimiques dans des fluides, selon les revendication 1 et 2, **caractérisée par le fait qu'**est utilisé un système pour le nettoyage de la cellule de réaction, des senseurs et des circuits de liquide entre les cycles analytiques successifs;

4. Une méthode pour le monitorage en continu de la présence de substances chimiques dans des fluides, selon les revendication 1 à 3, **caractérisée par le fait que** la Demande Biochimique d'Oxygène (DBO) peut être déterminée dans la cellule de réaction en utilisant la partie aliquote microbienne extraite depuis le chemostat et une électrode d'oxygène dissout situé dans la cellule de réaction.

5. Un appareil (Figure 1) pour le monitorage en continu de la présence de substances chimiques dans des fluides pour mener à bien la méthode de la revendication 1, **caractérisé par le fait qu'**il comprend :
a/ un système pour la culture continue des microorganismes utilisés, qui comprend :
• un chemostat situé dans un compartiment doté d'un thermostat
• un dépôt de liquide nutriment stérilisé dans un compartiment doté d'un thermostat
• un dispositif pour l'aération forcée dans le chemostat
• un dispositif pour la régulation indépendante de la température du chemostat
• un dispositif pour la régulation indépendante de la température du liquide nutriment
• des dispositifs pour l'injection du liquide nutriment et l'extraction de la partie aliquote microbienne
b/ un système pour les mesures analytiques basées sur les microorganismes cultivés, qui comprend :
• un dépôt d'un échantillon de référence (substrat de calibrage) situé dans un compartiment doté d'un thermostat
• une cellule de réaction située dans le compartiment doté d'un thermostat
• un dispositif pour la régulation indépendante de la température de la cellule de réaction
• un dispositif pour l'aération forcée de la cellule de réaction
• un dispositif pour la régulation indépendante de la température du dépôt de l'échantillon de référence
• un circuit de prélèvement de l'échantillon avec un filtre tangentiel
• des dispositifs pour l'injection des microorganismes cultivés, échantillon de référence et échantillon filtré
c/ un système pour le nettoyage du circuit du liquide.

6. Un appareil pour le monitorage en continu de la présence de substances chimiques dans des fluides, selon la revendication 5, **caractérisé par le fait que** la cellule de réaction inclut des senseurs spécifiques pour la détection des signaux physico-chimiques générés dans le métabolisme microbien par les substances chimiques intéressantes présentes dans l'échantillon.

7. Un appareil pour le monitorage en continu de la présence de substances chimiques dans des fluides, selon la revendication 5, **caractérisé par le fait que** l'on utilise un système pour le nettoyage de la cellule de réaction, des senseurs, et du circuit du liquide, qui comprend un dépôt de liquide de nettoyage situé dans le compartiment dont la température est régulée.

8. Un appareil pour le monitorage en continu de la présence de substances chimiques dans des fluides, selon la revendication 5, qui comprend un logiciel pour l'intégration et le traitement des signaux électriques générés par les senseurs.

9. Un appareil pour le monitorage en continu de la présence de substances chimiques dans des fluides, selon la revendication 5, qui comprend un microprocesseur et son logiciel pour le contrôle automatique de l'appareil, permettant la sélection par l'usager des paramètres correspondant au protocole analytique pour des applications spécifiques.

10. Un appareil pour le monitorage en continu de la présence de substances chimiques dans des fluides, selon la revendication 5, qui comprend un logiciel et un modem pour la transmission de données à travers une ligne téléphonique et pour le contrôle des paramètres du protocole analytique depuis un ordinateur à distance.
